(19) Europäisches Patentamt / European Patent Office / Office européen des brevets

(11) **EP 3 583 888 A1**

(12) **EUROPEAN PATENT APPLICATION**

(43) Date of publication:
**25.12.2019 Bulletin 2019/52**

(51) Int Cl.:
**A61B 5/00** *(2006.01)*     **A61B 5/107** *(2006.01)*

(21) Application number: **18178549.4**

(22) Date of filing: **19.06.2018**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.
5656 AE Eindhoven (NL)**

(72) Inventors:
• **DE HAAN, Gerard
  5656 AE Eindhoven (NL)**
• **WANG, Wenjing
  5656 AE Eindhoven (NL)**

(74) Representative: **de Haan, Poul Erik et al
Philips International B.V.
Philips Intellectual Property & Standards
High Tech Campus 5
5656 AE Eindhoven (NL)**

(54) **DEVICE, SYSTEM AND METHOD FOR IMAGE SEGMENTATION OF AN IMAGE OF A SCENE INCLUDING A SUBJECT**

(57) The present invention relates to a device, system and method for image segmentation of an image of a scene including a subject. To improve the segmentation, the device comprises a receiver (210) configured to receive electromagnetic radiation reflected from a scene including a subject, a polarization unit (220) configured to apply a first polarization on the received electromagnetic radiation to generate first polarized radiation and to apply a second polarization, which is different from the first polarization, on the received electromagnetic radiation to generate second polarized radiation, a sensor unit (230) configured to generate a first image from the first polarized radiation and a second image from the second polarized radiation, and a segmentation unit (250) configured to identify areas of different materials in the scene from a combination of the first and second images.

FIG.1

**Description**

FIELD OF THE INVENTION

The present invention relates to a device, system and method for image segmentation of an image of a scene including a subject. The present invention may particularly be used as a preliminary step of a method for determining at least one vital sign of a subject.

BACKGROUND OF THE INVENTION

Vital signs of a person, for example the heart rate (HR), the respiration rate (RR) or the (peripheral or pulsatile) blood oxygen saturation (SpO2; it provides an estimate of the arterial blood oxygen saturation SaO2), serve as indicators of the current state of a person and as powerful predictors of serious medical events. For this reason, vital signs are extensively monitored in inpatient and outpatient care settings, at home or in further health, leisure and fitness settings.

One way of measuring vital signs is plethysmography. Plethysmography generally refers to the measurement of volume changes of an organ or a body part and in particular to the detection of volume changes due to a cardio-vascular (or cardio-pulmonary) pulse wave traveling through the body of a subject with every heartbeat.

Photoplethysmography (PPG) is an optical measurement technique that evaluates a time-variant change of light reflectance or transmission of an area or volume of interest. PPG is based on the principle that the blood absorbs light more than the surrounding tissue, so variations in blood volume with every heartbeat affect the transmission or reflectance correspondingly. Besides information about the pulse rate (heart rate), a PPG waveform (also called PPG signal) can comprise information attributable to further physiological phenomena such as the respiration. By evaluating the transmittance and/or reflectance at different wavelengths (typically red and infrared), the blood oxygen saturation can be determined.

Conventional pulse oximeters (also called contact PPG device herein) for measuring the pulse rate and the (arterial) blood oxygen saturation of a subject are attached to the skin of the subject, for instance to a fingertip, earlobe or forehead. Therefore, they are referred to as 'contact' PPG devices. Although contact PPG is basically regarded as a non-invasive technique, contact PPG measurement is often experienced as being unpleasant and obtrusive, since the pulse oximeter is directly attached to the subject and the cables limit the freedom to move and might hinder a workflow.

Non-contact, remote PPG (rPPG) devices (also called camera-based devices or video health monitoring devices) for unobtrusive measurements have been proposed in the last decade. Remote PPG utilizes light sources or, in general, radiation sources, disposed at a distance from the subject of interest. Similarly, a detector, e.g. a camera or a photodetector, can be disposed at a distance from the subject of interest. Therefore, remote photoplethysmographic systems and devices are considered unobtrusive and well suited for medical as well as non-medical everyday applications.

Using the PPG technology, vital signs can be measured. Vital signs are revealed by minute light absorption changes in the skin caused by the pulsating blood volume, i.e. by periodic color changes of the human skin induced by the blood volume pulse. As this signal is very small and hidden in much larger variations due to illumination changes and motion, there is a general interest in improving the fundamentally low signal-to-noise ratio (SNR). There still are demanding situations, with severe motion, challenging environmental illumination conditions, or strict accuracy requirements, where an improved robustness and accuracy of the vital sign measurement devices and methods is required, particularly for the more critical healthcare applications.

Video health monitoring (to monitor or detect e.g. heart rate, respiration rate, SpO2, actigraphy, delirium, etc.) is a promising emerging field. Its inherent unobtrusiveness has distinct advantages for patients with fragile skin, or in need of long-term vital signs monitoring, such as NICU patients, patients with extensive burns, mentally-ill patients that remove contact-sensors, or COPD patients who have to be monitored at home during sleep. In other settings such as in a general ward or emergency room, the comfort of contactless monitoring is still an attractive feature.

Skin detection has a range of applications and often involves a form of color segmentation, or uses characteristic color variations of living skin over the cardiac cycle. There is a particular interest in automatic region of interest (ROI) detection for rPPG measurements for use in patient monitoring. Another potential application is in surveillance to reliably distinguish real and fake skin (e.g. masks). This topic is particularly relevant given the security risks involved when someone can successfully hide his/her identity or pretend to be someone else.

The problem with color-based segmentation methods is that they fail whenever the background contains skin-colored surfaces. This is even more common in the near infrared part of the spectrum, where skin reflection is very similar to that of bedding. Since patient monitoring preferably works in full darkness, no use can be made of reflection differences in the visible spectrum. Cardiac-induced color variations, on the other hand, may disappear if a patient suffers a heart attack. Hence, the color variation based feature loses the skin during the most critical event of the monitoring process.

In conclusion, there is a need for an improved device, system and method for image segmentation of an image of a scene including a subject leading to a more reliable segmentation even in case the background contains skin-colored surfaces. Further, there is a need to improve the determining of at least one vital sign of a subject to obtain results with higher reliability.

SUMMARY OF THE INVENTION

It is an object of the present invention to provide a device, system and method image segmentation of an image of a scene including a subject, by which desired image regions, e.g. skin regions, can be segmented with higher reliability.

In a first aspect of the present invention a device is presented comprising

- a receiver configured to receive electromagnetic radiation reflected from a scene including a subject,
- a polarization unit configured to apply a first polarization on the received electromagnetic radiation to generate first polarized radiation and to apply a second polarization, which is different from the first polarization, on the received electromagnetic radiation to generate second polarized radiation,
- a sensor unit configured to generate a first image from the first polarized radiation and a second image from the second polarized radiation, and
- a segmentation unit configured to identify areas of different materials in the scene from a combination of the first and second images.

In a further aspect of the present invention a system is presented comprising

- an illumination unit configured to illuminate a scene including a subject with unpolarized electromagnetic radiation or with polarized electromagnetic radiation, and
- a device as disclosed herein for image segmentation of an image of the illuminated scene.

In yet a further aspect of the present invention, there is provided a corresponding method.

Preferred embodiments of the invention are defined in the dependent claims. It shall be understood that the claimed method and system have similar and/or identical preferred embodiments as the claimed device, in particular as defined in the dependent claims and as disclosed herein.

The present invention is based on the idea that polarized light gets depolarized when reflected from a turbid medium like skin. The degree of depolarization depends on various parameters and provides another feature to distinguish between equally colored tissues. A segmentation using e.g. the local degree of depolarization in the image helps to solve the problem of skin detection in various applications in the health and patient monitoring domain.

Preferably, the scene including a subject is illuminated with unpolarized electromagnetic radiation or with polarized electromagnetic radiation (e.g. linearly, circular or elliptically polarized) radiation. In this case, a cross-polarizer in front of a first image sensor can suppress the specular reflection (DC and variations) significantly, leaving the PPG signal and the intensity variations. The other sensor, equipped with a parallel polarizer, is then modulated by the PPG signal, the specular and the intensity variations. If the specular reflection variations are not the strongest distortion, the intensity variations artifact can be suppressed by mixing both channels. If specular distortion is strongest, only the cross-polarized channel may be used, while a small fraction of the parallel channel may be subtracted to compensate for imperfections of the polarizers. Further, methods that fully automatically decide upon the optimal de-mixing of the PPG signal may be used.

In an embodiment said segmentation unit is configured to identify areas of different materials in the scene from a ratio or difference of the first and second images. This provide a good result for the segmentation.

In another embodiment said sensor unit is configured to generate the first and second images in a single wavelength channel or in two or more different wavelength channels. For instance, a monochromatic sensor, or an RGB sensor (like a conventional image sensor), or a sensor equipped with a filter array, e.g. a Bayer-filter, may be applied that provides three detection signal in three different color channels.

Using more than one wavelength channel may improve the segmentation. Accordingly, in a preferred embodiment said sensor unit is configured to generate the first and second images in two or more different wavelength channels and said segmentation unit is configured to identify areas of different materials in the scene from a combination of the first and second images per wavelength channel. In an alternative embodiment said sensor unit is configured to generate the first and second images in two or more different wavelength channels and said segmentation unit is configured to convert pixels of the respective first and second images into vectors with components of the two or more different wavelength channels, to normalize the vectors to unit length and to determine the cosine angle or inner product between the vectors of the two or more different wavelength channels in order to identify areas of different materials in the scene.

The device may further comprise a vital sign determination unit configured to select an area representing skin of the subject, to generate two or more detection signals in different wavelength channels from either the first polarized radiation or the second polarized radiation in the selected area, and to determine a vital sign from the two detection signals by combining the two detection signals. Hereby, the detection signals of different wavelengths are preferably all in the same polarization direction, particularly in the orthogonal polarization direction with respect to the polarization of the illumination light. This improves reliability of the determination of vital signs.

According to an embodiment said polarization unit is configured to apply the two different polarizations simultaneously and said sensor unit is configured to generate the two detection signals simultaneously. This embodiment may be implemented by use of a prism that splits the incoming radiation into to output portions of radiation having different polarization directions.

Alternatively, said polarization unit may be configured to apply the two different polarizations time-sequentially and said sensor unit may be configured to generate the two detection signals time-sequentially. Hereby, said polarization unit may be configured to alternate the polarization direction in time. This embodiment may be implemented by use of a polarizer that is able to change the polarization like an electrically controllable polarization filter.

Generally, as polarizer a polarization filter may be used. However, for this purpose, not only transmission filters can be employed, but reflectors and/or mirrors (e.g. polarization mirrors) may be used to achieve the same effect.

Good results are achieved by use of a polarization unit that is configured to apply a first polarization, which is orthogonal to the second polarization.

In another embodiment polarized illumination is used. Hereby, said polarization unit is configured to apply a first polarization which is parallel to or equivalent to (i.e. the same as; e.g. in case of circular polarization) the polarization direction of polarized electromagnetic radiation used for illuminating the skin region of the subject and a second polarization which is orthogonal or opposite (e.g. in case of circular polarization) to the polarization direction of the polarized electromagnetic radiation used for illuminating the skin region of the subject.

The device may further comprise a vital sign determination unit configured to select an area representing skin of the subject, to generate a first detection signal having a first polarization direction from the first polarized radiation in the selected area and a second detection signal having a second polarization direction, which is different from the first polarization direction, from the second polarized radiation in the selected area, and to determine a vital sign from the two detection signals by combining the two detection signals.

Further, said vital sign determination unit may be configured to determine a vital sign from the two detection signals by linearly combining the two detection signals by a weighted combination, wherein weights of said weighted combination are determined by blind signal separation, in particular by principal component analysis or independent component analysis, and by selecting a component channel of the combined detection signals according to a predetermined criterion. Such a blind signal separation method is e.g. described in WO 2017/121834 A1. The criterion may e.g. be the signal with the highest peak in the normalized corresponding spectrum, or the signal with the maximum skewness of the corresponding spectrum, etc. Generally, the detection signals may be from different wavelength channels, but preferably from the same polarization. It is also possible to use multiple wavelengths and different polarization direction and combine all of them.

Still further, in another embodiment said vital sign determination unit is configured to determine a vital sign from the two detection signals by linearly combining the two detection signals by a weighted combination using weights resulting in a pulse signal for which the products with the original detection signals equals the relative pulsatilities as represented by the respective signature vector, a signature vector providing an expected relative strength of the detection signal in the two original detection signals.

Generally, a PPG signal results from variations of the blood volume in the skin. Hence, the variations give a characteristic pulsatility "signature" when viewed in different spectral components of the reflected/transmitted light. This "signature" is basically resulting as the contrast (difference) of the absorption spectra of the blood and that of the bloodless skin tissue. If the detector, e.g. a camera or sensor, has a discrete number of color channels, each sensing a particular part of the light spectrum (wherein the parts may (partially) overlap and sensing a "part" does not necessarily mean all wavelength in the part contribute equally to the output), then the relative pulsatilities in these channels can be arranged in a "signature vector", also referred to as the "normalized blood-volume vector", PBV. It has been shown in G. de Haan and A. van Leest, "Improved motion robustness of remote-PPG by using the blood volume pulse signature", Physiol. Meas. 35 1913, 2014, which is herein incorporated by reference, that, if this signature vector is known, then a motion-robust pulse signal extraction on the basis of the color channels (or signals derived from these color channels) and the signature vector is possible. For the quality of the pulse signal, it is essential though that the signature vector is accurate, as otherwise the known methods mixes noise into the output pulse signal in order to achieve the prescribed correlation of the pulse vector with the normalized color channels as indicated by the signature vector.

Details of the PBV method and the use of the normalized blood volume vector (called "predetermined index element having a set orientation indicative of a reference physiological information") have also been described in US 2013/271591 A1, whose details are also herein incorporated by reference.

The characteristic wavelength-dependency of the PPG signal varies when the composition of the blood changes. Particularly, the oxygen saturation of the arterial blood has a strong effect on the light absorption in the wavelength range between 620nm and 780nm. This changing signature for different SpO2 values leads to relative PPG pulsatility that depends on the arterial blood oxygen saturation. This dependency can be used to realize a motion-robust remote SpO2 monitoring system that has been named adaptive PBV method (APBV) and is described in detail in M. van Gastel, S. Stuijk and G. de Haan, "New principle for measuring arterial blood oxygenation, enabling motion-robust remote monitoring", Nature Scientific Reports, Nov. 2016. The description of the details of the APBV method in this document is also herein incorporated by reference.

The PBV method gives the cleanest pulse signal when the PBV vector, reflecting the relative pulsatilities in the different wavelength channels is accurate. Since this vector depends on the actual SpO2 value, testing the PBV method with different PBV vectors, corresponding to a range of SpO2 values, the SpO2 value results as the one corresponding to the PBV vector giving the pulse-signal with the highest SNR.

The receiver of the proposed device may be configured in different ways, in particular to receive detection signals at different wavelengths, preferably depending on the kind of application and the system configuration. In general, the detection signals are selected from a wavelength interval between 300 nm and 1000 nm, in particular represent the wavelength portions corresponding to red, green and blue light. This is particularly used when the PPG signals are obtained from image signals acquired by a (e.g. conventional) video camera and when the above-mentioned principles of remote PPG are used for deriving one or more vital signs. In other embodiments infrared light may also be used in addition or instead of another color channel. For instance, for night-time applications one or more infrared wavelengths may be used in addition or alternatively.

The receiver may be configured as optical element, e.g. a lens, of an imaging unit, such as an optical sensor, a camera, e.g. a video camera, an RGB camera or web-cam.

Preferably, the illumination unit is configured to illuminate the skin region of the subject with unpolarized electromagnetic radiation or with polarized electromagnetic radiation within the wavelength range from 300 nm to 1000 nm.

According to an embodiment the illumination unit is configured to illuminate the skin region of the subject with linearly polarized electromagnetic radiation with a central wavelength in a wavelength range from 300 nm to 1000 nm.

In an embodiment, the energy of the emitted electromagnetic radiation is spread in a wavelength interval around said central wavelength, wherein said sensor unit comprises two sensor elements, a first sensor element being configured to generate one or two first detection sub-signal having a polarization direction, which is parallel to the polarization direction of the polarized electromagnetic radiation, and a second sensor element being configured to generate one or two second detection sub-signal having a polarization direction, which is orthogonal to the polarization direction of the polarized electromagnetic radiation, wherein a first detection sub-signal represents electromagnetic radiation in a first wavelength sub-interval of said wavelength interval at least partly below said central wavelength and a second detection sub-signal represents electromagnetic radiation in a second wavelength sub-interval of said wavelength interval at least partly above said central wavelength, and wherein said vital sign determination unit is configured to determine a vital sign from the detection sub-signals by combining the detection sub-signals.

According to an alternative embodiment the illumination unit is configured to illuminate the skin region of the subject with linearly polarized electromagnetic radiation with a central wavelength in a wavelength range from 300 nm to 1000 nm, wherein the energy of the emitted electromagnetic radiation is spread in a wavelength interval around said central wavelength, wherein said sensor unit comprises four sensor elements, wherein a first and second sensor element are configured to generate one or two first detection sub-signal having a polarization direction, which is parallel to the polarization direction of the polarized electromagnetic radiation, and a third and fourth sensor element are configured to generate one or two second detection sub-signal having a polarization direction, which is orthogonal to the polarization direction of the polarized electromagnetic radiation, wherein one of the first and second detection sub-signals represents electromagnetic radiation in a first wavelength sub-interval of said wavelength interval at least partly below said central wavelength and the other of the first and second detection sub-signal represents electromagnetic radiation in a second wavelength sub-interval of said wavelength interval at least partly above said central wavelength, and wherein said vital sign determination unit is configured to determine a vital sign from the detection sub-signals by combining the detection sub-signals.

The first and second wavelength sub-intervals are generally different. For instance, the first wavelength sub-interval is below said central wavelength and the second wavelength sub-interval of said wavelength interval is above said central wavelength. Both wavelength sub-intervals may, however, also overlap.

The proposed system may further comprise an output unit configured to output the vital sign. The output unit may e.g. be a user interface like a display, computer or loudspeaker. Still further, the proposed system may comprise a control unit configured to generate, based on the vital sign, an alarm control signal for controlling an alarm unit configured to issue an alarm and to output the generated alarm control signal.

BRIEF DESCRIPTION OF THE DRAWINGS

These and other aspects of the invention will be apparent from and elucidated with reference to the embodiment(s) described hereinafter. In the following drawings

Fig. 1 shows a schematic diagram of an embodiment of a system according to the present invention;
Fig. 2 shows a schematic diagram of a first embodiment of a device according to the present invention;
Fig. 3 shows a schematic diagram of a second embodiment of a device according to the present invention; and
Figs. 4A-4E show images of a scene illustrating segmentation by use of the ratio of a parallel-polarization image and a cross-polarization image.

DETAILED DESCRIPTION OF THE INVENTION

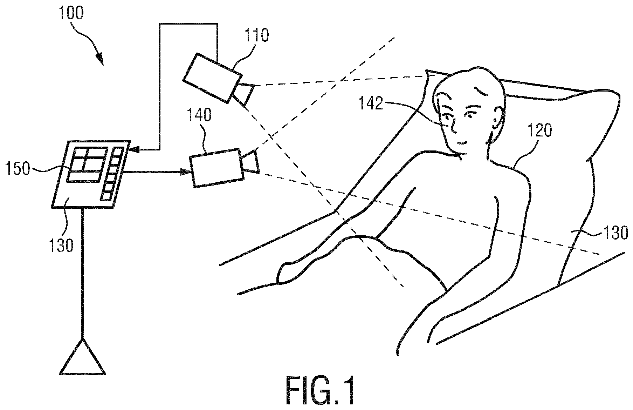

Fig. 1 shows a schematic diagram of an embodiment of a system 100 according to the present invention. The system 100 comprises an imaging unit 110 for receiving electromagnetic radiation reflected from a skin region of a subject 120. The system 100 further comprises a device 130 for image segmentation of an image of a scene including a subject and, optionally, for determining at least one vital sign of a subject from the received electromagnetic radiation. The subject 120, in this example a patient, lies in a bed 125, e.g. in a hospital or other healthcare facility, but may also be a neonate or premature infant, e.g. lying in an incubator, or person at home or in a different environment.

The system 100 may further comprise a light source 140 (also called illumination unit), such as a lamp, for illuminating a scene with unpolarized electromagnetic radiation or with polarized electromagnetic radiation (light). Said scene includes a region of interest, such as the skin of the patient's face (e.g. part of the cheek or forehead), and other non-skin areas, such as parts of the bed, clothing of the patient, etc. The emitted radiation may be light in a predetermined wavelength range or ranges (e.g. in the red, green and/or infrared wavelength range(s)). The radiation may be polarized with a predetermined (e.g. linear, circular or elliptical) polarization having a predetermined polarization direction, but may alternatively be non-polarized.

The light reflected from said region of interest 142 in response to said illumination is received by a receiver, e.g. the lens of the imaging unit 110 (e.g. a camera) or other optics in front of a sensor. In another embodiment no dedicated light source is provided, but ambient light is used for illumination of the subject 120. From the reflected light, only light in a number of desired wavelength ranges (e.g. green and red or infrared light, or light in a sufficiently large wavelength range covering at least two wavelength channels) may be detected and/or evaluated.

The device 130 may further be connected to an interface 150 for displaying the determined information and/or for providing medical personnel with an interface to change settings of the device 130, the imaging unit 110, the light source 140 and/or any other parameter of the system 100. Such an interface 150 may comprise different displays, buttons, touchscreens, keyboards or other human machine interface means.

A system 100 as illustrated in Fig. 1 may, e.g., be located in a hospital, healthcare facility, elderly care facility or the like. Apart from the monitoring of patients, the present invention may also be applied in other fields such as neonate monitoring, general surveillance applications, security monitoring or so-called live style environments, such as fitness equipment, a wearable, a handheld device like a smartphone, or the like. The uni- or bidirectional communication between the device 130 and the interface 150 may work via a wireless or wired communication channel. Other embodiments of the present invention may include an imaging unit, which comprises part or all of the device 130, which is not provided stand-alone, but integrated into the imaging unit 110, such as a camera.

Typically, the electromagnetic radiation is in the range of 400 nm to 1000 nm for pulse, respiration and blood oxygen saturation measurement, particularly in the range of 620 nm to 920 nm. This particular range is most suitable for SpO2 measurement and is attractive for unobtrusive monitoring during sleep (darkness), but if pulse or respiratory signals are required, the visible part of the spectrum may allow a higher quality (i.e. NIR is not necessarily the preferred option in all cases). The detection signals may be acquired by a photo-sensor (array) and/or using a video camera remotely sensing the subject's skin.

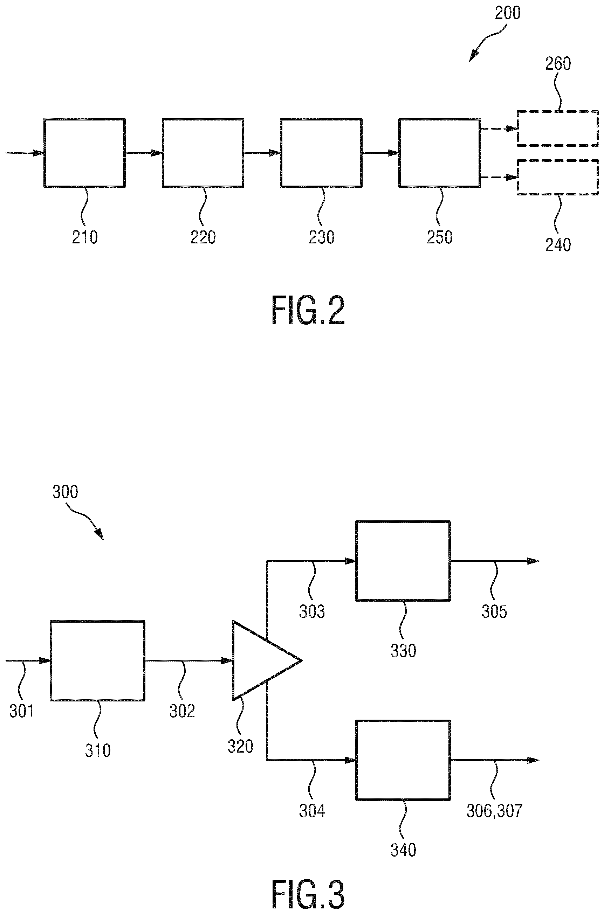

Fig. 2 shows a schematic diagram of a first embodiment of a device 200 according to the present invention for image segmentation of an image of a scene including a subject and, in this embodiment, for determining at least one vital sign of a subject. The device 200 may be integrated into the imaging unit 110, e.g. a camera, or may partly be integrated into or combined with the imaging unit 110 and partly be realized by the device 130, e.g. a processor or computer. It shall be noted that the device 200 is preferably used in the context of determining vital signs from electromagnetic radiation transmitted through or reflected from a skin area of a subject (rPPG), but other applications of the device 200 for image segmentation are in the field of general image processing where an image shall be segmented (e.g. in image processing of images from surveillance cameras (e.g. to distinguish skin from non-skin or skin-imitations)).

The device 200 comprises a receiver 210 configured to receive electromagnetic radiation reflected from a skin region of a subject. The receiver 210 may be an optical element, such as a lens of a camera that receives the radiation, in particular light in the desired wavelength range. The light reflected from the region of interest 121 in response to the illumination is received by the receiver 210.

In another embodiment no dedicated light source is provided, but ambient light is used for illumination of the subject 120. From the reflected light, only light in a number of desired wavelength ranges (e.g. green and red or infrared light, or light in a sufficiently large wavelength range covering at least two wavelength channels) may be detected and/or evaluated.

The device 200 further comprises a polarization unit 220 configured to apply a first polarization on the received electromagnetic radiation to generate first polarized radiation and to apply a second polarization, which is different from the first polarization, on the received electromagnetic radiation to generate second polarized radiation. The polarization unit 220 may e.g. comprise a prism or polarization filter(s). The first polarization may be parallel polarization and the second polarization may be cross polarization, which is orthogonal to the parallel polarization. However, other polarization directions and relationships of the polarizations are generally possible.

The device 200 further comprises a sensor unit 230 configured to generate a first image from the first polarized radiation and a second image from the second polarized radiation. The sensor unit may comprise two separate sensor elements, each generating one of the two images. The sensor unit (or each sensor element) may comprise a filter unit configured to filter the differently polarized radiation to obtain images in two or more wavelength channels.

The device 200 further comprises a segmentation unit 250 configured to identify areas of different materials in the scene from a combination of the first and second images.

The device 200 may hence be used for segmentation of images. The result of the segmentation may be used in various applications or may be directly issued, e.g. displayed on an (optional) screen 260. In one embodiment the device 200 may be used for an application that is directed to determining one or more vital signs. In such an application the device 200 may further comprise an (optional) vital sign determination unit 240 configured to determine a vital sign, or another device is using the result of the segmentation. The segmentation and the vital sign determination will be explained below in more detail.

The segmentation unit 250 may be comprised in a digital or analog processor or computer and/or may completely or partly be implemented in software and carried out on a computer. Some or all of the required functionality may also be implemented in hardware, e.g. in an application specific integrated circuit (ASIC) or in a field programmable gate array (FPGA). In an embodiment, the segmentation unit 250 may be part of the device 130 of the system 100. In another embodiment, the segmentation unit 250 is integrated into the imaging unit 110. The same holds for the optional vital sign determination unit 240.

To determine vital signs the present invention may exploit the observation that the light reflected from the skin has two components: the surface reflection component and the component that originates from scattering processes underneath the surface in the translucent skin tissue. The surface reflection undergoes single scattering event, preserving the polarization of the incident light. The scattered light, undergoes multiple reflections, causing the incident light to depolarize. The amount of depolarization can be expressed as:

$$P = \frac{I_p - I_c}{I_p + I_c} \tag{1}$$

where $I_p$, and $I_c$ are the intensity of the reflected light parallel (index p) and perpendicular (index c) to the incident light polarization. It is reasonable to assume that the original polarization of light is lost in biological perfused tissue, although the present invention works as well if partial polarization still exists.

Only the scattered component is actually modulated by the blood-volume changes that occur inside the tissue, while the surface (or specular) reflection component remains unmodulated. Therefore, the pulsatility modulated signal can be separated from the non-modulated signal by using light of a determined polarization to illuminate the subject. In other words, with polarized light, only the scattered reflections become visible when using orthogonal (cross) polarizer in front of the camera. With a parallel oriented polarizer in front of the camera, the surface reflection dominates the image.

For the remote PPG, since the scattered component contains the information about the variations of the blood volume, only the scattered light may be considered to extract the PPG signal. As the reflected photons that have penetrated into the skin have been scattered multiple times inside the skin, they have lost their initial polarization direction. This is in contrast with the specularly reflected light that keeps the polarization of the incident light. By using polarized illumination, a cross-polarizer in front of the imaging sensor suppresses specularly reflected light. Since the specular (surface) reflection is typically not desired, a sensor with a parallel polarizer has therefore not been used so far.

In an embodiment it is proposed to simultaneously, or time-sequentially, image with different polarizers, e.g. with both specular (parallel-polarized) and scattered (crossed-polarized) light. The resulting channels (different polarizations) lead to different mixtures of the wanted PPG signal and motion artefacts, which can be combined to extract a motion-robust PPG signal.

An embodiment of the system uses a time-sequential depolarization setup, in which the system comprises one camera and one illumination unit and in which the polarization direction is changed in time, i.e. at different times different polarization directions are applied. In an alternative embodiment a similar effect can be achieved by a dual-camera setup simultaneously, in which one camera has a parallel polarization direction with the illumination unit and the other camera has cross-polarization direction.

Fig. 3 shows a schematic diagram of a second embodiment of a device 300 according to the present invention. Polarized illumination (polarized light) is emitted (by the illumination unit 140; see Fig. 1) to a scene including one or more skin areas of the subject as well as non-skin areas, e.g. of the background, clothes, etc. Light 301 reflected from the skin is received by the receiving element 310, e.g. an optical lens. The received light 302 is guided to an optical element 320, e.g. a polarization unit, that separates the cross and parallel polarized beams 303, 304 and projects them on separate sensors 330, 340 (together e.g. forming a sensor unit), each optionally comprising sensor elements with a different sensitivity to parts of the radiation spectrum, to detect different wavelength channels. Alternatively, the sensors 330, 340 may all have the same sensitivity to the radiation spectrum. In an exemplary embodiment the sensors 330, 340 are both equipped with a pixelated (Bayer) filter to acquire the different wavelength channel in parallel for each polarization direction. However, various alternatives exist.

In this embodiment, the polarization unit 320 (e.g. a prism or polarization filter) is generally configured to apply a first polarization on the received electromagnetic radiation 302 to generate first polarized radiation 303 and to apply a second polarization, which is different from the first polarization, or no polarization on the received electromagnetic radiation 302 to generate second polarized or non-polarized radiation 304. Further, the sensor unit comprising the sensors 330, 340 is generally configured to derive at least one first image 305 from the first polarized radiation 303 and to derive one second image 306 from the second polarized or non-polarized radiation 304.

In further embodiments it is provided that each sensor 330, 340 derives two or three images in two or three different wavelength channels, and or that the light beams 303, 304 are split further, separate wavelength selective filters are used in each path to a separate sensor.

Hence, in an embodiment of the device a polarized light source is used to illuminate a scene including a subject with a visible skin surface. An imaging sensor is viewing the scene through a polarizer that switches polarization direction (e.g. using a liquid crystal material) between parallel and orthogonal (cross) orientations compared with the polarization direction of the light source. If cost is less of a concern, two separate sensors can be used to simultaneously acquire the cross- and parallel channels, as shown in Fig. 3. Two successive images now view the same scene (apart from little motion that may be present in case of time-sequential acquisition) through different polarizers. Depending on the depolarization characteristics of the various surfaces the relative strength of the reflection in the cross- and parallel channels will differ. In case of pure specular reflection, the cross-polarized image will show a completely dark surface, while the parallel channel will show a bright area. In case of complete depolarization, the parallel and the cross-polarized channels will show the same brightness.

Hence, a feature helping to classify different materials can be built, using the two polarization channels. Simple examples of a feature are the ratio of the two images or their difference. Consequently, for different materials, even if they have the same total reflection (brightness, color), such a feature helps to segment the image into similar (material) groups of pixels.

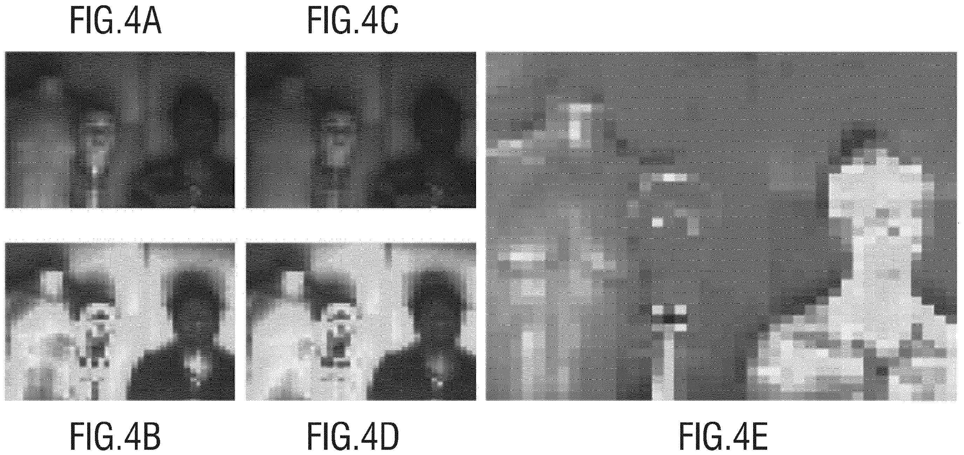

Figs. 4A-4E illustrate this basic scenario. In particular, segmentation based on a single cross-parallel ratio feature is illustrated that is computed on the red channel only of a color image. The scene contains a human face, a doll-face and a torso. Fig. 4A shows an image sensed after the parallel polarizer. Fig. 4B shows the corresponding red channel image. Fig. 4C shows an image sensed after the cross-polarizer. Fig. 4D shows the corresponding red channel image. Fig. 4E shows the cross-parallel ratio image in the red channel.

Although in this case the segmentation seems easy, because the face is darker than the skin-similar objects, it should be borne in mind that the feature is blind to brightness as it is eliminated in the ratio. This is a best case that suffices for illustration, though generally a single wavelength may not suffice to reliably differentiate between skin and non-skin, and better results are possible using two or more (e.g. three) color channels.

The degree of depolarization may further depend on the wavelength used. Therefore, an advantageous embodiment of the device uses a multi-wavelength image sensor. For instance, an RGB-visible light video camera may be used to collect a parallel and a cross-polarized image for three wavelength channels (red, green and blue). Pixels are converted to vectors with components R, G, and B and normalized to unit length for both polarization channels. Now, the cosine-angle (inner-product) between the vectors taken from the two polarization channels may be used as a feature (in this case the only feature) to segment the image as shown in Fig. 4. The facial skin of the subject can clearly been segmented, even though skin-similar objects (torso and doll-face) occur in the image.

The multi-wavelength approach may be very interesting to skin detection, particularly using NIR (near-infrared) wavelengths. For patient monitoring, discriminating between skin and textile (bedding) is a crucial element in remote photoplethysmography. A NIR camera with three channels (760 nm, 800 nm and 900 nm) and corresponding polarizers (preferably for the NIR wavelength range) may be used to substantially improve segmentation.

In all above cases, Blind Source Separation (BSS) techniques, like Principle Component Analysis (PCA), or Independent Component Analysis (ICA) may be used to extract the independent signals, as e.g. described in the above cited paper of G. de Haan and A. van Leest, "Improved motion robustness of remote-PPG by using the blood volume pulse signature". PCA requires the relative energy of the different components to be sufficiently different, but is often simpler and more robust than ICA, which however can deal with independent signals with equal energy in the mix. Whichever BSS method is being used, two independent signals are obtained from the two sensor signals. To choose the PPG signal, a common strategy is to find the one that is most periodic. To decide this, often a Fourier transform on a time-lapse of the signals is done and the selection is based on this transform, e.g. by choosing the signal of which the spectrum has the lowest entropy, or the spectrum with the highest skewness, or the spectrum with the highest frequency peak after normalizing the spectrum.

Another preferred option is to use the PBV methods as e.g. described as well in the above cited paper of G. de Haan and A. van Leest, "Improved motion robustness of remote-PPG by using the blood volume pulse signature", to find the motion-robust PPG signal. To describe the procedure in this case, normalized signals are written from both sensors in a time window as vectors, $C_p$ and $C_c$, that are combined into a matrix, $C_n=[C_p, C_c]$, and the extracted pulse (PPG) signal is written as S, which is a weighted sum of the two sensor signals:

$$S = W \, C_n$$

The PBV-method obtains the mixing coefficients using the prior knowledge regarding the relative strength of the pulse in the two camera-channels. In our case, we expect the pulse to be equally strong in both channels, i.e. Pbv= [1, 1], provided both channels are normalized by the DC value of the cross-polarized channel. The best results are obtained if the band-passed filtered versions of the two polarized channels are used.

According to this method the known $P_{bv}$ vector is used to discriminate between the pulse signal and distortions. Given that the relative amplitude of the pulse signal at in cross- and polarized channel is given by $P_{bv}$, the weights, $W_{PBV}$, are searched that give a pulse signal S, for which the correlation with the two polarized channels $C_p$ and $C_c$ equals $P_{bv}$

$$\vec{S} C_n^T = k \vec{P}_{bv} \Leftrightarrow \vec{W}_{PBV} C_n C_n^T = k \vec{P}_{bv},$$

and consequently the weights determining the mixing are determined by

$$\vec{W}_{PBV} = k \vec{P}_{bv} Q^{-1} \text{ with } Q = C_n C_n^T,$$

and the scalar k can be determined such that $W_{PBV}$ has unit length (or another fixed length).

In other words, the weights indicate how the detection signals should be (linearly) combined in order to extract a pulse signal from the detection signals. The weights are unknown and need to be computed/selected.

The signature vector (PBV vector) represent the given (known or expected) relative pulsatilities in different wavelength channels (i.e. the detection signals), caused by the absorption spectrum of the blood and the penetration of light into the skin (if photons are more absorbed by blood, a volume change of blood leads to a larger signal than when the blood is nearly transparent). With this knowledge, and the observed data (i.e. the detection signals) the weights (e.g. a weight vector) can be determined. The resulting weights are data dependent, i.e. depend on the detection signals.

Since the pulse signal has a different ratio AC/DC (this is also called the relative signal strength / pulsatility) in each wavelength channel, it can be seen that the spectrum shows the pulse peak in the spectrum with different peak values for the different colors. This spectrum is the result of a Fourier analysis, but it basically means that if a sinusoid having the pulse frequency is correlated (multiplied) with the detection signals (RGB in the example, NIR-wavelengths for SpO2), exactly the peak values in the spectrum are obtained, which by definition are called the signature vector (PBV vector): these peak values are the relative strength of the normalized amplitudes of the pulse signal in the different detection signals.

The consequence of this is that a clean pulse signal S can be obtained (assuming the pulse signal is the result of a weighted sum of the detection signals), using this prior knowledge (i.e. the signature vector). One option to do this is to compute an inversion of a covariance matrix Q of the normalized detection signals $C_n$. Hence, the weights W to linearly mix the detection signals in order to extract the pulse signal S can be computed from the covariance matrix of the detection signals in the current analysis window (Q, which is data dependent, i.e. changes continuously over time), using the constant signature vector PBV.

The above described methods can be applied on detection signals that have been acquired using contactless sensors. By way of example, the present invention can be applied in the field of healthcare, e.g. unobtrusive remote patient monitoring, surveillance, security monitoring and so-called lifestyle environments, such as fitness equipment or the like. Applications may include monitoring of oxygen saturation (pulse oximetry), pulse rate, blood pressure, cardiac output, changes of blood perfusion, assessment of autonomic functions, respiration, and detection of peripheral vascular diseases. The present invention can e.g. be used for rapid and reliable pulse detection of a critical patient, for instance during automated CPR (cardiopulmonary resuscitation). The system can be used for monitoring of vital signs of neonates with very sensitive skin e.g. in NICUs and for patients with damaged (e.g. burnt) skin, but may also be more convenient than contact sensors as used in the general ward, and offer better solutions for motion robustness. Finding the relevant skin area automatically is currently one of the bottlenecks. Another potential application is in surveillance to reliably distinguish real and fake skin (e.g. masks).

While the invention has been illustrated and described in detail in the drawings and foregoing description, such illustration and description are to be considered illustrative or exemplary and not restrictive; the invention is not limited to the disclosed embodiments. Other variations to the disclosed embodiments can be understood and effected by those skilled in the art in practicing the claimed invention, from a study of the drawings, the disclosure, and the appended claims.

In the claims, the word "comprising" does not exclude other elements or steps, and the indefinite article "a" or "an" does not exclude a plurality. A single element or other unit may fulfill the functions of several items recited in the claims. The mere fact that certain measures are recited in mutually different dependent claims does not indicate that a combination of these measures cannot be used to advantage.

A computer program may be stored/distributed on a suitable non-transitory medium, such as an optical storage medium or a solid-state medium supplied together with or as part of other hardware, but may also be distributed in other forms, such as via the Internet or other wired or wireless telecommunication systems.

Any reference signs in the claims should not be construed as limiting the scope.

**Claims**

1. Device for image segmentation of an image of a scene including a subject, said device comprising:

   - a receiver (210) configured to receive electromagnetic radiation reflected from a scene including a subject,
   - a polarization unit (220) configured to apply a first polarization on the received electromagnetic radiation to generate first polarized radiation and to apply a second polarization, which is different from the first polarization, on the received electromagnetic radiation to generate second polarized radiation,
   - a sensor unit (230) configured to generate a first image from the first polarized radiation and a second image from the second polarized radiation, and
   - a segmentation unit (250) configured to identify areas of different materials in the scene from a combination of the first and second images.

2. Device as claimed in claim 1,
   wherein said segmentation unit (250) is configured to identify areas of different materials in the scene from a ratio or difference of the first and second images.

3. Device as claimed in any one of the preceding claims,
   wherein said sensor unit (230) is configured to generate the first and second images in a single wavelength channel or in two or more different wavelength channels.

4. Device as claimed in any one of the preceding claims,
   wherein said sensor unit (230) is configured to generate the first and second images in two or more different wavelength channels and
   wherein said segmentation unit (250) is configured to identify areas of different materials in the scene from a combination of the first and second images per wavelength channel.

5. Device as claimed in any one of the preceding claims,
   wherein said sensor unit (220) is configured to generate the first and second images in two or more different wavelength channels and
   wherein said segmentation unit (250) is configured to convert pixels of the respective first and second images into vectors with components of the two or more different wavelength channels, to normalize the vectors to unit length and to determine the cosine angle or inner product between the vectors of the two or more different wavelength channels in order to identify areas of different materials in the scene.

**6.** Device as claimed in any one of the preceding claims,
further comprising a vital sign determination unit (240) configured to select an area representing skin of the subject, to generate two or more detection signals in different wavelength channels from either the first polarized radiation or the second polarized radiation in the selected area, and to determine a vital sign from the two detection signals by combining the two detection signals.

**7.** Device as claimed in any one of the preceding claims,
wherein said polarization unit (220) is configured to apply the two different polarizations simultaneously and said sensor unit (230) is configured to generate the two images simultaneously.

**8.** Device as claimed in any one of the preceding claims,
wherein said polarization unit (220) is configured to apply the two different polarizations time-sequentially, in particular to alternate the polarization direction in time, and said sensor unit (230) is configured to generate the two images time-sequentially.

**9.** Device as claimed in any one of the preceding claims,
wherein said polarization unit (220) is configured to apply a first polarization which is orthogonal to the second polarization.

**10.** Device as claimed in any one of the preceding claims,
wherein said polarization unit (220) is configured to apply a first polarization which is parallel or equivalent to the polarization direction of polarized electromagnetic radiation used for illuminating the skin region of the subject and a second polarization which is orthogonal or opposite to the polarization direction of the polarized electromagnetic radiation used for illuminating the skin region of the subject.

**11.** Device as claimed in any one of the preceding claims,
further comprising a vital sign determination unit (240) configured to select an area representing skin of the subject, to generate a first detection signal having a first polarization direction from the first polarized radiation in the selected area and a second detection signal having a second polarization direction, which is different from the first polarization direction, from the second polarized radiation in the selected area, and to determine a vital sign from the two detection signals by combining the two detection signals.

**12.** Device as claimed in claim 6 or 11,
wherein said vital sign determination unit (240) is configured to determine a vital sign from the two detection signals by linearly combining the two detection signals by a weighted combination, wherein weights of said weighted combination are determined by blind signal separation, in particular by principal component analysis or independent component analysis, and by selecting a component channel of the combined detection signals according to a predetermined criterion.

**13.** Device as claimed in claim 6 or 11,
wherein said vital sign determination unit (240) is configured to determine a vital sign from the two detection signals by linearly combining the two detection signals by a weighted combination using weights resulting in a pulse signal for which the products with the original detection signals equals the relative pulsatilities as represented by the respective signature vector, a signature vector providing an expected relative strength of the detection signal in the two original detection signals.

**14.** System for image segmentation of an image of a scene including a subject, said system comprising:

- an illumination unit (140) configured to illuminate a scene including a subject with unpolarized electromagnetic radiation or with polarized electromagnetic radiation, and
- a device (200) as claimed in claim 1 for image segmentation of an image of the illuminated scene.

**15.** Method for image segmentation of an image of a scene including a subject, said method comprising:

- receiving electromagnetic radiation reflected from a scene including a subject,
- applying a first polarization on the received electromagnetic radiation to generate first polarized radiation and to apply a second polarization, which is different from the first polarization, on the received electromagnetic radiation to generate second polarized radiation,

- generating a first image from the first polarized radiation and a second image from the second polarized radiation, and
- identifying areas of different materials in the scene from a combination of the first and second images.

FIG.1

FIG.2

FIG.3

FIG.4C

FIG.4A

FIG.4E

FIG.4D

FIG.4B

Europäisches
Patentamt

European
Patent Office

Office européen
des brevets

# EUROPEAN SEARCH REPORT

Application Number

EP 18 17 8549

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 836 872 A (KENET ROBERT O [US] ET AL) 17 November 1998 (1998-11-17) <br> * column 16, line 10 * <br> * column 18, line 48 * <br> * column 22, line 61 - column 23, line 7 * | 1,15 | INV. <br> A61B5/00 <br> A61B5/107 |
| Y | US 2015/272489 A1 (KIRENKO IHOR OLEHOVYCH [NL] ET AL) 1 October 2015 (2015-10-01) <br> * paragraphs [0012], [0013], [0028], [0036], [0037], [0051], [0052], [0053], [0056], [0057]; claim 1; figures 1,2 * | 1-15 | |
| A | US 2016/120482 A1 (KIRENKO IHOR OLEHOVYCH [NL] ET AL) 5 May 2016 (2016-05-05) <br> * paragraphs [0034], [0037], [0038]; figure 1 * | 1 | |
| Y | GARCIA MISSAEL ET AL: "Biologically inspired imaging sensors for multi-spectral and polarization imagery", PROCEEDINGS OF SPIE; [PROCEEDINGS OF SPIE ISSN 0277-786X VOLUME 10524], SPIE, US, vol. 10655, 14 May 2018 (2018-05-14), pages 106550C-106550C, XP060105333, DOI: 10.1117/12.2305119 ISBN: 978-1-5106-1533-5 <br> * pages 1-3; figures 1,2,5 * | 1-15 | |

TECHNICAL FIELDS
SEARCHED      (IPC)

A61B

-/--

The present search report has been drawn up for all claims

| Place of search | Date of completion of the search | Examiner |
|---|---|---|
| The Hague | 14 December 2018 | Pameijer, Robert |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

**Europäisches Patentamt**
**European Patent Office**
**Office européen des brevets**

# EUROPEAN SEARCH REPORT

Application Number

EP 18 17 8549

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | THOMAS W CRONIN ET AL: "Multichannel spectrometers in animals", BIOINSPIRATION & BIOMIMETICS, INSTITUTE OF PHYSICS PUBLISHING, BRISTOL, GB, vol. 13, no. 2, 9 February 2018 (2018-02-09), page 21001, XP020324867, ISSN: 1748-3190, DOI: 10.1088/1748-3190/AAA61B [retrieved on 2018-02-09] * page 12, column 2; figures 9,10 * | 1,15 | |
| A | GARCIA MISSAEL ET AL: "Live demonstration: A 1300 x 800, 700 mW, 30 fps spectral polarization imager", 2015 IEEE INTERNATIONAL SYMPOSIUM ON CIRCUITS AND SYSTEMS (ISCAS), IEEE, 24 May 2015 (2015-05-24), page 1911, XP033183576, DOI: 10.1109/ISCAS.2015.7169044 [retrieved on 2015-07-27] | 1 | |
| A | TIM YORK ET AL: "Live demonstration: Material detection via an integrated polarization imager", CIRCUITS AND SYSTEMS (ISCAS), 2011 IEEE INTERNATIONAL SYMPOSIUM ON, IEEE, 15 May 2011 (2011-05-15), page 1990, XP031998041, DOI: 10.1109/ISCAS.2011.5937983 ISBN: 978-1-4244-9473-6 * page 1 * | 1 | |

-/--

The present search report has been drawn up for all claims

| Place of search | Date of completion of the search | Examiner |
|---|---|---|
| The Hague | 14 December 2018 | Pameijer, Robert |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 18 17 8549

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | VIKTOR GRUEV ET AL: "Material detection with a CCD polarization imager", APPLIED IMAGERY PATTERN RECOGNITION WORKSHOP (AIPR), 2010 IEEE 39TH, IEEE, 13 October 2010 (2010-10-13), pages 1-7, XP031861115, DOI: 10.1109/AIPR.2010.5759710 ISBN: 978-1-4244-8833-9 * equations 1-6; figures 2,3,9; table 1 * | 1,2 | |
| A | GARCIA MISSAEL ET AL: "A 1300 x 800, 700 mW, 30 fps spectral polarization imager", 2015 IEEE INTERNATIONAL SYMPOSIUM ON CIRCUITS AND SYSTEMS (ISCAS), IEEE, 24 May 2015 (2015-05-24), pages 1106-1109, XP033183363, DOI: 10.1109/ISCAS.2015.7168831 [retrieved on 2015-07-27] * equations 1-6; page 1106, column 1, lines 6,7, paragraph 3; figures 2-4 * | 1,2 | |
| A | YONGQIANG ZHAO ET AL: "Design and performance analysis of infrared micro-polarizer array", 2015 34TH CHINESE CONTROL CONFERENCE (CCC), TECHNICAL COMMITTEE ON CONTROL THEORY, CHINESE ASSOCIATION OF AUTOMATION, 28 July 2015 (2015-07-28), pages 4574-4580, XP033201934, DOI: 10.1109/CHICC.2015.7260347 [retrieved on 2015-09-11] * equations 1-5; figures 1,2,4,8-10 * | 1,2 | TECHNICAL FIELDS SEARCHED (IPC) |

-/--

The present search report has been drawn up for all claims

| Place of search | Date of completion of the search | Examiner |
|---|---|---|
| The Hague | 14 December 2018 | Pameijer, Robert |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 3 of 7

Europäisches
Patentamt

European
Patent Office

Office européen
des brevets

## EUROPEAN SEARCH REPORT

Application Number

EP 18 17 8549

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LI SHUYI ET AL: "Fabrication and evaluation of aluminum nano-wire grid polarizer array in two different structure types", 2017 IEEE INTERNATIONAL CONFERENCE ON MANIPULATION, MANUFACTURING AND MEASUREMENT ON THE NANOSCALE (3M-NANO), IEEE, 7 August 2017 (2017-08-07), pages 91-94, XP033315797, DOI: 10.1109/3M-NANO.2017.8286340 [retrieved on 2018-02-07] * figure 2 * | 1,15 | |
| A | ZHANG JIEYUN ET AL: "A novel smoothness-based interpolation algorithm for division of focal plane Polarimeters", 2017 IEEE INTERNATIONAL SYMPOSIUM ON CIRCUITS AND SYSTEMS (ISCAS), IEEE, 28 May 2017 (2017-05-28), pages 1-4, XP033155982, DOI: 10.1109/ISCAS.2017.8050355 [retrieved on 2017-09-25] * figure 1 * | 1,15 | |
| A | PARK HYUNSUNG ET AL: "Polarization-resolved imaging using elliptical silicon nanowire photodetectors", 2014 CONFERENCE ON LASERS AND ELECTRO-OPTICS (CLEO) - LASER SCIENCE TO PHOTONIC APPLICATIONS, THE OPTICAL SOCIETY, 8 June 2014 (2014-06-08), pages 1-2, XP032707288, DOI: 10.1364/CLEO_SI.2014.STH4I.5 [retrieved on 2014-12-16] * figures 1,2 * | 1,15 | TECHNICAL FIELDS SEARCHED (IPC) |

-/--

The present search report has been drawn up for all claims

| Place of search | Date of completion of the search | Examiner |
|---|---|---|
| The Hague | 14 December 2018 | Pameijer, Robert |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | VAN GASTEL MARK ET AL: "Motion Robust Remote-PPG in Infrared", IEEE TRANSACTIONS ON BIOMEDICAL ENGINEERING, IEEE SERVICE CENTER, PISCATAWAY, NJ, USA, vol. 62, no. 5, 1 May 2015 (2015-05-01), pages 1425-1433, XP011578781, ISSN: 0018-9294, DOI: 10.1109/TBME.2015.2390261 [retrieved on 2015-04-17] * equations 1-8; page 1426, column 2 * | 5,6,13 | |
| Y | US 2017/202633 A1 (LIU YANG [US]) 20 July 2017 (2017-07-20) | 8 | |
| A | * paragraph [0070] * | 1,7 | |
| Y | US 2016/162728 A1 (ARAI TOSHIYA [JP] ET AL) 9 June 2016 (2016-06-09) * paragraphs [0071], [0072], [0074], [0090] * | 6,10,11 | |
| Y | SUN YU ET AL: "Photoplethysmography Revisited: From Contact to Noncontact, From Point to Imaging", IEEE TRANSACTIONS ON BIOMEDICAL ENGINEERING, IEEE SERVICE CENTER, PISCATAWAY, NJ, USA, vol. 63, no. 3, 1 March 2016 (2016-03-01), pages 463-477, XP011599999, ISSN: 0018-9294, DOI: 10.1109/TBME.2015.2476337 [retrieved on 2016-02-18] | 12 | TECHNICAL FIELDS SEARCHED (IPC) |
| A | * page 470, lines 10, 19 * * page 471 * | 4 | |

-/--

The present search report has been drawn up for all claims

| Place of search | Date of completion of the search | Examiner |
|---|---|---|
| The Hague | 14 December 2018 | Pameijer, Robert |

Europäisches
Patentamt

European
Patent Office

Office européen
des brevets

**EUROPEAN SEARCH REPORT**

Application Number

EP 18 17 8549

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DE HAAN G ET AL: "Improved motion robustness of remote-PPG by using the blood volume pulse signature", PHYSIOLOGICAL MEASUREMENT, INSTITUTE OF PHYSICS PUBLISHING, BRISTOL, GB, vol. 35, no. 9, 27 August 2014 (2014-08-27), pages 1913-1926, XP020269523, ISSN: 0967-3334, DOI: 10.1088/0967-3334/35/9/1913 [retrieved on 2014-08-27] * page 1915 * | 5,12 | |
| A | Xavier Gonsalves ET AL: "A Review of Processing Techniques for Non- invasive Photoplethysmographic Extraction of Health Parameters", International Journal of Advanced Research in Electrical Electronics and Instrumentation Engineering (An ISO Certified Organization, 1 February 2014 (2014-02-01), pages 2320-3765, XP055130669, Retrieved from the Internet: URL:http://ijareeie.com/upload/2014/februa ry/13A_A-Review.pdf [retrieved on 2014-07-21] * page 7156 * | 12 | |

-/--

TECHNICAL FIELDS
SEARCHED (IPC)

The present search report has been drawn up for all claims

| Place of search | Date of completion of the search | Examiner |
|---|---|---|
| The Hague | 14 December 2018 | Pameijer, Robert |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt
European
Patent Office
Office européen
des brevets

**EUROPEAN SEARCH REPORT**

Application Number

EP 18 17 8549

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | SUBRAMANIAM NITYA ET AL: "Natural Material Segmentation and Classification Using Polarisation", 8 June 2011 (2011-06-08), INTERNATIONAL CONFERENCE ON SIMULATION, MODELING, AND PROGRAMMING FOR AUTONOMOUS ROBOTS,SIMPAR 2010; [LECTURE NOTES IN COMPUTER SCIENCE; LECT.NOTES COMPUTER], SPRINGER, BERLIN, HEIDELBERG, PAGE(S) 468 - 475, XP047427219, ISBN: 978-3-642-17318-9 | 1,15 | |
| A | NITYA SUBRAMANIAM ET AL: "Detection of skin lesions using diffuse polarisation", 2010 17TH IEEE INTERNATIONAL CONFERENCE ON IMAGE PROCESSING (ICIP 2010); 26-29 SEPT. 2010; HONG KONG, CHINA, IEEE, PISCATAWAY, NJ, USA, 26 September 2010 (2010-09-26), pages 3021-3024, XP031813294, ISBN: 978-1-4244-7992-4 | 1,15 | |

TECHNICAL FIELDS
SEARCHED      (IPC)

The present search report has been drawn up for all claims

| Place of search | Date of completion of the search | Examiner |
|---|---|---|
| The Hague | 14 December 2018 | Pameijer, Robert |

EPO FORM 1503 03.82 (P04C01)

## ANNEX TO THE EUROPEAN SEARCH REPORT
## ON EUROPEAN PATENT APPLICATION NO.

EP 18 17 8549

This annex lists the patent family members relating to the patent documents cited in the above-mentioned European search report.
The members are as contained in the European Patent Office EDP file on
The European Patent Office is in no way liable for these particulars which are merely given for the purpose of information.

14-12-2018

| Patent document cited in search report | | Publication date | Patent family member(s) | | Publication date |
|---|---|---|---|---|---|
| US 5836872 | A | 17-11-1998 | AU 5524990 A<br>US 5016173 A<br>US 5836872 A<br>WO 9013091 A1 | | 16-11-1990<br>14-05-1991<br>17-11-1998<br>01-11-1990 |
| US 2015272489 | A1 | 01-10-2015 | CN 106170242 A<br>EP 3125745 A1<br>JP 2017512576 A<br>US 2015272489 A1<br>WO 2015150106 A1 | | 30-11-2016<br>08-02-2017<br>25-05-2017<br>01-10-2015<br>08-10-2015 |
| US 2016120482 | A1 | 05-05-2016 | CN 107106017 A<br>EP 3212059 A1<br>JP 2017532151 A<br>US 2016120482 A1<br>WO 2016066724 A1 | | 29-08-2017<br>06-09-2017<br>02-11-2017<br>05-05-2016<br>06-05-2016 |
| US 2017202633 | A1 | 20-07-2017 | CN 106029000 A<br>EP 3107476 A1<br>US 2017202633 A1<br>WO 2015126466 A1 | | 12-10-2016<br>28-12-2016<br>20-07-2017<br>27-08-2015 |
| US 2016162728 | A1 | 09-06-2016 | CN 105407799 A<br>EP 3028632 A1<br>JP 6343612 B2<br>JP WO2015015793 A1<br>US 2016162728 A1<br>WO 2015015793 A1 | | 16-03-2016<br>08-06-2016<br>13-06-2018<br>02-03-2017<br>09-06-2016<br>05-02-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2017121834 A1
- US 2013271591 A1

**Non-patent literature cited in the description**

- **G. DE HAAN ; A. VAN LEEST.** Improved motion robustness of remote-PPG by using the blood volume pulse signature. *Physiol. Meas.,* 1913, vol. 35
- **M. VAN GASTEL ; S. STUIJK ; G. DE HAAN.** New principle for measuring arterial blood oxygenation, enabling motion-robust remote monitoring. *Nature Scientific Reports,* November 2016